Europäisches Patentamt

European Patent Office

Office européen des brevets

(19)

(11) Publication number: **0 308 717 B1**

(12) **EUROPEAN PATENT SPECIFICATION**

(45) Date of publication of patent specification: **30.12.92**   (51) Int. Cl.5: **A61K 39/42**

(21) Application number: **88114517.1**

(22) Date of filing: **06.09.88**

(54) Method of treating viral encephalitis.

(30) Priority: **22.09.87 US 99531**

(43) Date of publication of application:
**29.03.89 Bulletin  89/13**

(45) Publication of the grant of the patent:
**30.12.92 Bulletin  92/53**

(84) Designated Contracting States:
**AT BE CH DE ES FR GB GR IT LI LU NL SE**

(56) References cited:
**EP-A- 0 158 317**

**BIOLOGICAL ABSTRACTS, vol. 60, 15 August
1975, Philadelphia, PA (US); M.NARITA et al.,
no. 18789**

(73) Proprietor: **CLINICAL BIOTECHNOLOGIES,
INC.
300 West 11th Street
Kansas City Missouri 64105(US)**

(72) Inventor: **Stout, Robert L.
10521 Bradshaw Street
Overland Park Kansas 66214(US)**

(74) Representative: **UEXKÜLL & STOLBERG Paten-
tanwälte
Beselerstrasse 4
W-2000 Hamburg 52(DE)**

Rank Xerox (UK) Business Services

**Description**

Background of the Invention

1. Field of the Invention

The present invention is broadly concerned with a method for the treatment of encephalitis which provides both an ammelioration of symptoms and a cure for the disease. More particularly, it is concerned with such a method wherein an immunostimulating biologic is administered in vivo, such biologic being identified and designated by ATCC Accession No. 40105, and being fully described in U.S. Patent 4,572,834

2. Description of the Prior Art

Vaccines have been in use since Edward Jenner (1749-1823) first recognized that people exposed to non-virulent strains of microorganisms could be protected against infection by the related virulent strains. His vaccination of patients with exudate of cowpox sores provided those same patients with partial protection against smallpox. Numerous vaccines have subsequently been prepared and used in both humans and animals. Immunization has been accomplished by vaccination with a suspension of live, attenuated, or killed organisms or specific protein, glycoprotein or surface material from various bacteria, rickettsiae, or viruses. These would include vaccines against anthrax, rabies, typhoid, cholera, smallpox, measles, mumps, pertussis, plague, and polio. The vaccine in each case has been intended to provide protection against a specific pathogen. While vaccination may provide long term specific protection it may or may not produce short term immunosystem modulation. The immune system responds to challenge via vaccination or infection by increased activity. This may result in the short term production of antigen non-specific immunoregulatory modulators such as interferon, interleukins, and other various lymphokines.

The rationale for a system that becomes active only in response to a specific challenge is simple. If the immune system operated at a high pitch of activity all the time, it would age prematurely and no longer provide the systematic protection it was evolved to deliver. Another possible complication with an unusually active immune system could be the initiation of auto-immune reactions in which the system starts acting against itself, resulting in the destruction of normal tissue. Arthritis is exemplary of this particular condition.

Increased activity of the immune system by virtue of improper regulation could furthermore result in an inability to elicit an appropriate response. As an example, a massive response to a splinter in a finger would be completely inappropriate, just as no response to a major infection would be equally inappropriate.

Immunomodulation may provide a regulatory-directed approach at self-healing, particularly with respect to such intractable diseases as cancer. For this to occur two minimal criteria must be met. The immune system must be sufficiently intact to respond to the regulator(s), and secondly, the system under appropriate stimulation must have T-cells, B-cells and Natural Killer cells present that are capable of responding to the target antigen(s) or cell(s). In such an instance, however, a nonspecific immunomodulator could potentially serve to "turn on" the immune system and initiate healing.

A number of immunomodulators have previously been isolated and described and these appear to belong to one of three general groups, namely the interferons, the interleukins and the corticosteroids and leukotrienes.

The interferons are a family of glycoproteins normally produced in response to a viral infection and are produced by leukocytes and fibroblasts. There are three main types, alpha, beta and gamma. They have molecular weights in the range of 15,000 to 40,000 daltons. Recently they have been produced by recombinant DNA techniques. The interferons have been found to be specie restricted and are normally only effective in specie that produced it.

The second group of immunomodulators are the interleukins. They are a family of glycoproteins that are produced by white cells. It is believed that the lymphokines cause the activation of Natural Killer cells and B-cells, and act in the initiation and propagation of the specific sequences of cellular interactions that are now recognized as the immune response. These growth promoters and activators participate in the generation of immunoreactive cells. The lymphokines are antigen non-specific in that they activate all T and B cells in anticipation of the presentation of an antigen or a cell. The apparent molecular weights are in the range of 15,000 - 50,000 daltons. To date, treatment protocols for cancer patients involving interleukins have been of an in vitro character, i.e., blood is withdrawn from the patient, treated in vitro, and returned to the patient. This is a relatively cumbersome process and is subject to all of the typical problems attendant to in vitro, as opposed to in vivo, procedures.

The third type of regulators are the corticosteroids and the leukotrienes, which act as regulators in inflammation. When produced in atypical amount, the leukotrienes can cause immediate type hypersensitivity and anaphylaxis. They are principally oxygenated products of archidonic acid. In contrast the corticosteroids and leukotrienes are small molecular weight compounds in comparison to the interleukins and interferons.

All of the immunological regulators produced commercially are extracts or concentrates of tissue culture fluids from mitogen stimulated lymphoid and myleloid cells. The exceptions to this are interferons and IL-2 which have been produced by genetic engineering. The other immunological modulators that have been artificially produced are the interferons. The difficulty with each of these production methods is that the individual reagents being produced do not reproduce the plethoric effect seen in vivo. No practical method has heretofore been discovered to produce a plurality of the immunoregulators together in the same proportions as they would normally be produced in response to infection or cancer in situ. Additionally, the cost of prior known production methods has been considerable, thereby further limiting the utility of immunomodulators.

Encephalitis in man and animals has long been a serious disease. In many cases, the mortality experience with encephalitis is very high, particularly in equine encephalitis and human encephalitis in third world countries. Clinical chemical treatment for viral, bacterial and fungal encephalitis is generally limited by the blood/brain barrier. The meniges (the dura mater, pia mater and arachnoid) are a set of membranes that envelop the brain and spinal chord and physically separate these from the rest of the body. The meniges provide selective permeability to solutes present in blood and lymph. For example, the meniges allow glucose to readily access the brain while completely excluding the formed elements of blood, white and red cells. While it is well known that red and white cells are excluded from the brain, less well understood is the fact that many small solutes are also excluded.

For a drug to be effective in the treatment of encephalitis, it must therefore pass through the meniges and into the brain. In practice, it has been extremely difficult to find drugs having the desired antiviral, antifungal and/or antibacterial activity which can also cross the blood/brain barrier. As a consequence, past treatment of encephalitis has primarily involved treatment merely of the symptoms of the disease, rather than the cause.

## Summary of the Invention

The principal object of the present invention is to provide a method for the treatment of viral, bacterial or fungal encephalitis through the use of a non-toxic biologic of the type described in U.S. Patent No. 4,572,834. Broadly speaking, the method of the invention comprises administration of a purified or partially purified immunostimulating biologic to an in vivo subject. The biologic serves to increase the number and activity of Natural Killer cells, and also concomitantly reduces the number of suppressor cells. The biologic has other more broadly based antiviral activity. However, the biologic demonstrates no direct antiviral activity in the absence of white blood cells.

In preferred forms of the invention, the immunostimulant is prepared by injecting a virus into an animal, and permitting the injected animal to react to the presence of the virus for a period of time for developing in the animal's blood serum significant concentrations of the desired biologic. The specifically preferred method for obtaining the biologic is set forth in full detail below. However, it is to be understood that other methods of obtaining the biologic may well be more economical and direct, e.g., such as by genetic engineering.

In terms of in vivo human treatment modalities, the preferred methods of treatment will be by intravenous injection of the immunostimulant biologic in either purified or partially purified form; but it is also believed that other routes of administration will be applicable, such as intramuscular, subcutaneous, and intradermal injections.

The presently preferred method of obtaining the biologic involves injecting a virus such as a parvovirus into an animal, and permitting the injected animal to react to the presence of the virus for a period of time in order to develop in the animal's blood serum biologic in sufficient quantity and/or activity that 50 microliters of the animal's serum, when added to an in vitro human white blood cell culture containing 2-4 x $10^5$ white blood cells and followed by 3 days incubation at 37° C. under a 5% $CO_2$/95% air atmosphere, will give rise to at least about a 50% increase in Natural Killer cells in the biologic-supplemented cell culture, as compared with an otherwise identical and identically cultured in vitro cell culture having added thereto 50 microliters of serum from a normal animal of the same species as the injected animal. The final step in the method involves recovering serum from the injected animal which contains the desired biologic.

In particularly preferred forms the animal in question is a goat, and the virus injected is a Parvovirus of

3

a type which is immunosuppressive in a normal permissive host animal (e.g., a cat or dog) for the Parvovirus. Thus, in the presently preferred method of the invention, use is made of a normally immunosuppressive virus in a non-permissive host for the virus; quite surprisingly however, this serves to develop an immunostimulative biologic in the injected animal's serum which has the salutory effects outlined above.

In other forms, the injected animal can be selected from the group consisting of goats, horses, sheep, rabbits and monkeys, with the period of time after injection of the virus being at least about one month.

The injected and immunized animal's serum can be used in relatively crude form, such as after only conventional ammonium sulfate fractionation. In other instances, however, the fractionated serum is subjected to further isolation procedures, in order to substantially purify the biologic.

In actual practice, a number of test goats are normally immunized with Parvovirus, and the serum of these goats is tested after an appropriate time (e.g., about 3 months) for the presence of biologic using the standard outlined above. Those animals which are positives, i.e., their serum exhibits the biologic in the manner defined, are then bled and their serum, containing the biologic, is recovered.

## Brief Description of the Drawings

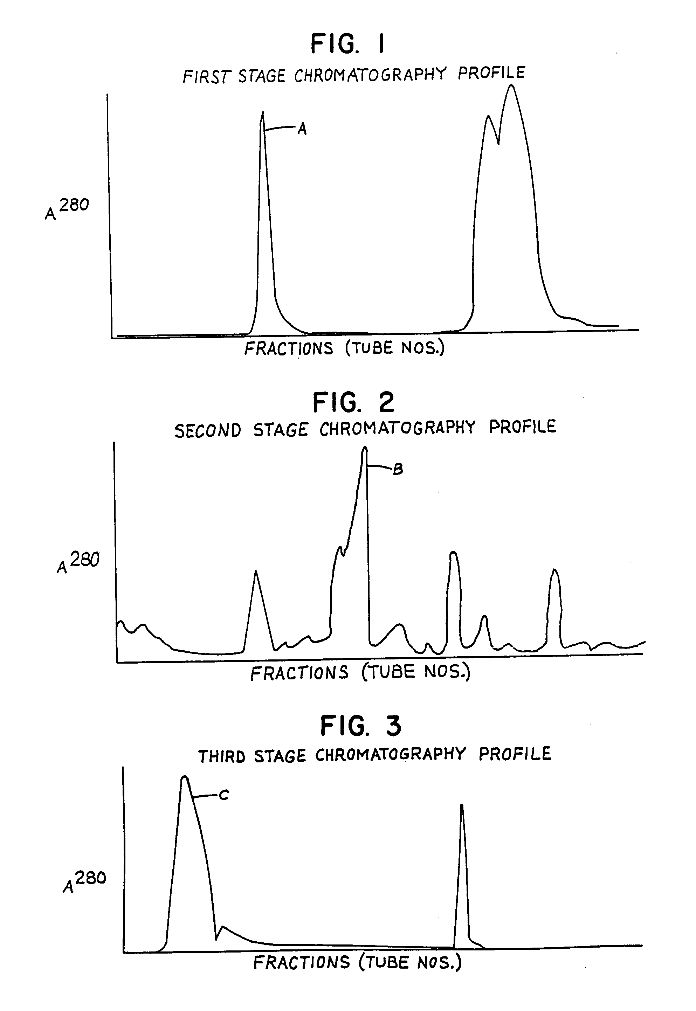

Figures 1-3 are respectively chromatography profiles developed as a result of first stage, second stage and third stage column chromatographies of ammonium sulfate fractionated Parvovirus-immunized positive goat serum in accordance with the preferred method of producing the biologic of the invention as disclosed in Example I.

## Description of the Preferred Embodiments

The following examples set forth the most preferred method of producing biologic used in the method of the invention.

## EXAMPLE I

This Example sets forth the preferred procedures for the production and recovery of the biologic of the present invention.

## Goat Vaccination

A total of five normal goats were initially hypervaccinated with Parvovirus in multiple sites on their dorsal rear halves, using a total of 1 cc. of commercially available Feline Pan Leukopenia vaccine (Dellen Laboratories, Inc., Westwood, Maine). Thereafter, at intervals of about one and two months after the initial injection, each goat was again injected with 1 cc. total quantities of Canine Origin Parvovirus vaccine (Fromm Laboratories, Inc., Grafton, Wisconsin or Duramune Vaccine, Temple, Texas). At the end of about three months from the date of the initial injection, a sample of whole blood was withdrawn from the carotid artery of the neck of each goat, and the samples treated as hereinafter described.

## Goat Plasma Preparation

The goat blood samples were separately centrifuged (about 500 ml. at a time) using a fixed angle rotor centrifuge operated at 2000 rpm for 45 minutes to pellet the red blood cells. The plasma was then removed from the packed red cells in each sample, and volumetrically measured. An absorbance reading (280 nm) of each plasma sample diluted 1:50 with PBS (pH 7.5) was then taken for record purposes.

## Serum Preparation

## Reagents Employed:

1.0 molar calcium Chloride
14.7 gm $CaCl_2 2H_2O$ in 100 ml distilled water
Saline
9 gms Sodium Chloride in 100 ml distilled water

## Procedure:

Use 1.0 ml of 1. molar calcium chloride for each 100 ml of plasma.

1. The required amount of calcium chloride was added to each measured plasma sample in a beaker.

2. The respective plasma/calcium chloride mixtures were then stirred gently and thereafter each beaker was placed in a coldbox overnight.

3. The clotted plasma in each beaker was cut up into small pieces.

4. Each mixture was then poured into a respective centrifuge tube, and the tubes were spun 10,000 rpm for 30 minutes.

5. Serum was then carefully removed from the clot at the bottom of each of the tubes to give separate serum samples.

6. For each sample the serum volume and an absorbance reading at 280 nm was taken and recorded.

Biologic Determination

An in vitro assay was next performed on the serum samples derived from each of the test goats, in order to determine which of the goats produced the desired biologic in sufficient quantity. Normal human white cells were first cultured in RPMI-1640 media (a standard tissue culture media produced by K. C. Biological of Lenexa, Kansas) supplemented with 10% fetal calf serum. Fifty microliters of each of the goat test serums produced as described previously were then added to respective cultures each containing 2-4 x $10^5$ cultured human white blood cells in a total volume of 0.2 ml. The cultures, with goat serum added, were then incubated in an atmosphere of 5% $CO_2$/95% air at 37° C. for 3 days. Each separate culture was then stained with two types of fluorescent-labeled monoclonal antibodies respectively specific against T-helper cells (CD4) (OKT-4 monoclonal antibody sold by Ortho Diagnostic of Raritan, New Jersey) and Natural Killer cells (CD 16) (LEU-7 monoclonal antibody sold by The Becton-Dickinson Co. of Mountain View, California). The stainings were performed following the supplier's directions, and after about one hour, the stained samples were analyzed using a flow cytometry device, namely a model 50H cytofluorograph (Ortho Instruments, Westwood, Mass.) to count Natural Killer cells in each culture. The test animal serums were compared against a stained and counted control sample comprising cultured human white blood cells and 50 microliters of normal goat serum prepared as outlined previously. Those cultures which exhibited about a 50% increase in the number of Natural Killer cells, as compared with the control, were considered positives for the biologic of the invention. In this specific test three of the five goat serum samples gave positive results; one sample gave about a 50% increase, whereas the other two samples gave an increase on the order of 125%.

The above described in vitro determination assay is used throughout the purification and isolation protocol in order to identify which of the separated fractions in each instance contained the desired biologic.

The three positive serum samples were each further treated as described below (the procedure for only a single serum sample is set forth, but the method of treating all the samples was the same). In this example, the respective positive serum samples were treated separately; however, such samples could be pooled if desired.

Ammonium Sulfate Fractionation

Reagents Employed:

Ammonium Sulfate (Sigma S-5182, Sigma Chemical Co., St. Louis, Missouri)
.01m Sodium Phosphate (pH 7.6)

Procedure:

1. 24.3 gm of ammonium sulfate was weighed out for each 100 ml of serum to be fractionated.

2. The serum sample was placed in a beaker and stirred. During stirring small amounts of ammonium sulfate were added, making sure that all was dissolved before adding more ammonium sulfate; this procedure was continued until the entire amount of ammonium sulfate was added to the sample. The sample was then stirred gently for another 30 minutes.

3. The serum sample was then placed in a centrifuge tube and spun at 10,000 rpm, for 30 minutes, whereupon supernatant was carefully removed from the pellet. The supernatant serum fraction was tested for the presence of the desired biologic using the in vitro cell culture assay described above under "Biologic Determination" and was found to be negative; this supernatant was therefore discarded.

4. The centrifuged pellet from step 3 was then redissolved in .01m sodium phosphate (pH 7.6), with the

5

final volume of redissolved pellet being the same amount as that of original serum volume.

5. Steps 2 and 3 were then repeated for the serum sample, in order to effect another separation; again, the in vitro determination assay confirmed that the supernatant did not contain the desired biologic.

6. The pellet was then redissolved to one-half of the original serum volume, using .01 M sodium phosphate (pH 7.6).

7. The resuspended material sample was then placed into a section of 12,000 mw cut-off dialysis tubing.

8. The sample was then dialyzed for 24 hours against .01 M sodium phosphate (pH 7.6), using three changes of 4 liters each in the dialysis.

9. The sample was then removed from the dialysis tubing and put into a centrifuge tube.

10. The tube was next spun at 10,000 rpm for 30 minutes.

11. The supernatant from the centrifuge tube was then removed and the volume measured and recorded; another in vitro determination assay as described above was performed on the supernatant, to confirm that it did contain the desired biologic.

12. The absorbance at 280 nm for the supernatant was taken and recorded.

13. The supernatant material resulting from the fractionation of the sample was stored in a sterile bottle.

First Stage Chromatography (using DE-52 anion exchange resin, Whatman, Inc., Clifton, N. J.)

1. An appropriate quantity of DE-52 resin was swelled and equilibrated in .01 molar Sodium Phosphate, pH 7.6.

2. A column (4.4 cm x 83 cm) was packed with swelled DE-52 resin. The column was washed with 2 liters of .01 M $NaH_2PO_4$ (pH 7.6) at 75 cm pressure.

3. 40 ml of the ammonium sulfate fractionated serum from the sample were loaded onto the DE-52 column at the same pressure as the wash. The material was then eluted from the column with .01 M $NaH_2PO_4$ (pH 7.6) wash.

4. Liquid from the column was collected in separate tubes (150 drops/tube--approximately 5.0 ml) until all protein fractions were completely eluted. About 1.0 liter of .01 molar $NaH_2PO_4$ was passed through the column.

5. The column was then washed with 1.5 liters of .01 molar $NaH_2PO_4$ containing .5 m NaCl (pH 7.6) to remove bound material from the column. Liquid from the columns was collected in separate tubes (150 drops/tube) until the buffer was expended.

6. The absorbance at 280 nm was taken for all tubes collected. A plot of absorbance ($A^{280}$) vs. tube number was prepared to locate the peaks. This graph is reproduced as Fig. 1.

7. The material within tubes located in each separate peak off the plot were then pooled and assayed using the described in vitro determination assay.

8. The pooled material from the first peak (peak A of Fig. 1) was found to contain the biologic of the invention. This material was concentrated by placing the material into a section of dialysis tubing and tying off the ends; the tubing was then placed into a tub of polyethylene glycol (20,000 mw, Sigman Chemical Co., St. Louis, Missouri) and concentrated to about 10% of the original pooled volume. The tubing was then rinsed well with water.

9. The pooled material within the tubing was then dialyzed for 24 hours against 0.01 $\underline{M}$ $KH_2PO_4$ pH 6.8. Three changes of 4 liters were used to remove polyethylene glycol and salt.

10. The dialyzed material was then removed from tubing and the volume measured. Absorbance readings at 280 nm were taken and recorded along with volume.

Second Stage Chromatography (using S-Sepharose cation exchange chromatography resin Pharmacia Fine Chemicals, Sigma Chemical Co., St. Louis, Missouri)

1. A column (1.0 x 90 cm) was packed with S-Sepharose resin and then equilibrated with 2 liters of 0.01 M $KH_2PO_4$ pH 6.8 at flow rate of 0.1 ml/minute.

2. One (1) ml of the pooled and dialyzed material from the first concentrated peak (peak A, Fig. 1), from the DE-52 column was loaded onto the S-Sepharose columns. The column was then developed with a linear gradient of sodium chloride 0 to 0.5 M in 0.01 M $KH_2PO_4$ pH 6.8. The flow rate was maintained at 0.1 ml with a high pressure liquid chromatograph pump. The gradient was developed over six hundred minutes.

3. Sixty drop fractions were collected (approximately 1.5 ml) in separate tubes. The absorbance at 280 nm was read on a Varian DMS90 for each fraction. A plot of $A^{280}$ vs. fraction number was prepared; this plot is reproduced as Figure 2.

4. The material within the tubes located in each peak were pooled and concentrated with the polyethylene glycol protocol as set forth in steps 7 and 8 of the first stage chromatography procedure. The material present in each fraction was then tested for stimulatory activity using the in vitro determination assay.

5. The pooled material from the largest peak (peak B, Fig. 2) was found to contain the biologic. This material was then dialyzed for 24 hours using dialysis tubing against 3 changes of 4 liters of PBS (0.01 M $KH_2PO_4$ containing 0.15 M NaCl, pH 7.5).

6. The dialyzed material was then removed from the tubing and the volume measured and recorded. An absorbance ($A^{280}$) was then taken on the dialyzed material, and the reading recorded.

Third Stage Gel Chromatography (using CM Blue AFFI gel cation exchange resin, Bio-Rad Corp., Richmond, California)

1. A column (1.5 cm x 38 cm) was packed and equilibrated with the CM Blue AFFI gel, and the column was washed with 2 liters of .01 M $K_2HPO_4$ containing .15 M NaCl, pH 7.25, at 45 cm pressure.

2. Two milligrams of material in 1 ml of liquid from the largest peak off the S-Sepharose column (peak B, Fig. 2) was then loaded onto the column.

3. The column was then developed with .01 M $K_2HPO_4$ containing .15 M NaCl (pH 7.25) until all peaks were eluted, using approximately 150-200 ml PBS for the column. Liquid off the column was collected in 90 drops fractions, about 2 ml in separate tubes.

4. The column was again washed with 200 ml of .01 M $K_2HPO_4$ containing .5 m NaCl (pH 7.25 to further elute the bound material; liquid from the column was collected in 90 drops fractions in separate tubes.

5. Absorbance readings at 280 nm were taken for all tubes and a plot of $A^{280}$ vs tube number from both elutions was prepared; this plot is reproduced as Fig. 3. Material within each peak was pooled, and these were assayed using the in vitro determination assay.

6. Material from the first peak (peak C, Fig. 3) was found to contain most of the biologic; this material was concentrated with polyethylene glycol as described above in the first stage chromatography procedure.

7. The concentrated material was dialyzed for 24 hours against PBS, using three changes of 4 liters each.

8. The material was next removed from dialysis tubing. Absorbance at 280 nm was read and recorded.

9. The material was filtered using a 0.2 um filter and stored.

As is evident from the foregoing, after a determination is made for the positive serum samples (i.e., those containing adequate amounts of biologic) successive fractionation and separation techniques may be employed to obtain the relatively purified biologic material, with appropriate assays being performed on separated fractions in order to ensure that the biologic is retained throughout. The final biologic product comprises proteinaceous component(s) which have not to date been completely characterized. However, the product is presently believed to be an interleukin protein or proteins having an apparent molecular weight in the range of 100,000 to 120,000 daltons; the components also appear to be glycosylated. A sample of the purified biologic made in accordance with the present invention has been deposited with the American Type Culture Collection; such sample has been accorded accession number 40105. Obviously, however, there is no desire to be bound to any preliminary or partial characterizations, and such are offered only in an effort to disclose all presently available pertinent information.

While the above described separation and isolation procedures are in many cases preferred, it should be understood that the biologic product derived from the methods of the invention may be usable without all of the aforementioned purification procedures.

EXAMPLE II

Antiviral Activity Against Venezuelan Equine Encephalitis Virus

The immunostimulant was tested for its effectiveness for treatment of viral encephalitis. CD-1 Swiss mice were infected with Venezuelan Equine Encephalitis Virus by intraperitoneal injection of 1.3 plaque forming units of virus. The mice were broken into two groups, treated and controls. The test group was treated by a single injection of 0.2 ml containing 20 or 100 ug of immunostimulant. The test animals were divided into four groups. All four groups were treated after they were infected. Group one was treated the day they were infected. Group two, three and four were treated on day two, four and six respectively. The data for the study is presented in Table I.

TABLE I

| Efficacy of IMC as an Anti-Venezuelan Equine Encephalitis Virus Agent In Vivo[1] | |
| --- | --- |
| Treatment | # Dead/# Treated |
| 20 ug IMC day 0 | 10/10 ( 8.9 +/- 1.7)[2] |
| 100 ug IMC day 0 | 7/10 ( 7.6 +/- 1.5) |
| 20 ug IMC day 2 | 7/10 ( 8.3 +/- 1.5) |
| 100 ug IMC day 2 | 10/10 ( 9.5 +/- 1.8) |
| 20 ug IMC day 4 | 6/10 ( 9.0 +/- 1.8) |
| 100 ug IMC day 4 | 6/10 ( 8.3 +/- 1.0) |
| 20 ug IMC day 6 | 5/10 ( 10.8 +/- 2.0) |
| 100 ug IMC day 6 | 7/10 ( 9.4 +/- 0.5) |
| Untreated | 8/9 ( 8.9 +/- 1.5) |

[1] CD-1 Swiss mice were infected with Venezuelan Equine Encephalitis Virus by I.P. injection of 1.3 pfu. Animals were treated with (0.2 ml) IMC once by injection on day of infection or 2, 4, or 6 days after infection.

[2] Average day of death +/- standard deviation.

The greatest degree of survivability was found with the animals treated at four or six days after infection, (6/10, 6/10 and 5/10, 7/10) respectively.

By comparison, the average day of death of the untreated control mice was 8.9 days, just 2.9 days after the animals in the fourth group were treated.

## Claims

1. Use of the immunostimulating biologic identified and designated by the ATCC Accession No. 40105 for the preparation of a medicament for the in vivo treatment of encephalitis.

2. Use according to claim 1 for the preparation of a medicament for the treatment of mammals and particularly of humans.

## Patentansprüche

1. Verwendung der immunstimulierenden biologischen Substanz, welche durch die ATCC Nr. 40105 identifiziert und gekennzeichnet ist, zur Herstellung eines Arzneimittels für die in vivo Behandlung von Encephalitis.

2. Verwendung nach Anspruch 1 zur Herstellung eines Arzneimittels zur Behandlung von Säugern, insbesondere von Menschen.

## Revendications

1. Usage de la substance biologique immunostimulante identifiée et désignée par le numéro d'ordre ATCC 40105 pour la préparation d'un médicament pour le traitement in vivo de l'encéphalite.

2. Usage selon la revendication 1, pour la préparation d'un médicament pour le traitement de mammifères et notamment de l'homme.

# FIG. 1

### FIRST STAGE CHROMATOGRAPHY PROFILE

$A^{280}$

A

FRACTIONS (TUBE NOS.)

# FIG. 2

### SECOND STAGE CHROMATOGRAPHY PROFILE

$A^{280}$

B

FRACTIONS (TUBE NOS.)

# FIG. 3

### THIRD STAGE CHROMATOGRAPHY PROFILE

$A^{280}$

C

FRACTIONS (TUBE NOS.)